# EUROPEAN PATENT SPECIFICATION

(11) **EP 0 477 774 B1**
(45) Date of publication and mention of the grant of the patent: **24.04.1996**
(21) Application number: 91115936.6
(22) Date of filing: 19.09.1991
(51) Int. Cl.: A23L 1/025, A23C 19/00, A23C 9/12, A21D 8/06, A23L 1/218, A23L 1/105

(54) **Method of producing brewed, matured or fermented foods using vibrations**
Verfahren zur Herstellung von gebrauten, gereiften oder fermentierten Nahrungsmitteln mittels Schwingungen
Méthode pour la préparation d'aliments brassés, mûris ou fermentés en utilisant des vibrations

(30) Priority: 28.09.1990 JP 258962/90
(43) Date of publication of application: 01.04.1992
(73) Proprietor: BODYSONIC KABUSHIKI KAISHA, Shinjuku-ku, Tokyo (JP)
(72) Inventor: Komatsu, Akira, c/o Bodysonic Kabushiki Kaisha, Tokyo (JP)
(74) Representative: Lehn, Werner, Dipl.-Ing.

(56) References cited:
- DATA BASE WPIL/DERWENT AN=89-212411 29 DW8929 Derwent Publications Ltd., London, GB. & RO-A-96464 INTR. UTILAJ. ALIMENT. 28.02.89
- DATA BASE WPIL/DERWENT AN=90-174638 23 DW9023 Derwent Publications Ltd., London, GB. & JP-A-2113847 ISEKI AGRIC.MACH.MFG.KK 26.4.90
- DATA BASE WPI/DERWENT AN=80-08851C 05 DW8005 Derwent Publications Ltd., London, GB. & SU-A-663359 BREAD BAKING IND. 28.05.79
- DATA BASE WPI/DERWENT AN=72-77908T 49 DW7249 Derwent Publications Ltd., London, GB. & SU-A-333918 TIKHANE NM.
- DATA BASE WPIL/DERWENT AN=83-732924 32 DW8332 Derwent Publications Ltd., London, GB. & SU-A-960247 WINE GROWING PROD. RES. 23.9.82
- DATA BASE WPI/DERWENT AN=80-80421C 45 DW8045 Derwent Publications Ltd., London, GB. & SU-A-724567 FERMENT PRODUCTS 31.03.80
- DATA BASE WPI/DERWENT AN=80-58432C 33 DW8033 Derwent Publications Ltd., London, GB.& SU-A-706442 MASLOBOEV G. YA. 30.12.79

## Description

### BACKGROUND OF THE INVENTION

### Field of the Invention

The present invention is related to that of EP-A-0 469 585, which concerns a method of producing brewed, matured, or fermented foods (hereinafter to be referred to as "fermented foods"), wherein the method is designed to attain a reduction of the brewing, maturing, or fermenting time (hereinafter called a "fermenting time") and additionally making improvements on the taste of such foods.

### Description of Prior Art

Among foods, there are numerous items produced by fermentation. Such fermented foods are produced with a process of fermentation matched with their respective conditions. Now, the yeasts which are used for these foods are microorganisms. It has been verified through experiments that these microorganisms have more animated activities when vibrations are given to them and that they have more vigorous propagating power than at a time when vibrations are not given to them. Thus, it can be considered to utilize vibrations for the process of fermentation. The simplest vibration-generating apparatus available for this purpose is a mechanical apparatus using a cam or the like. Also, as a generally employed apparatus which generates vibrations by an electrical process, such an apparatus using ultrasonic waves may be cited.

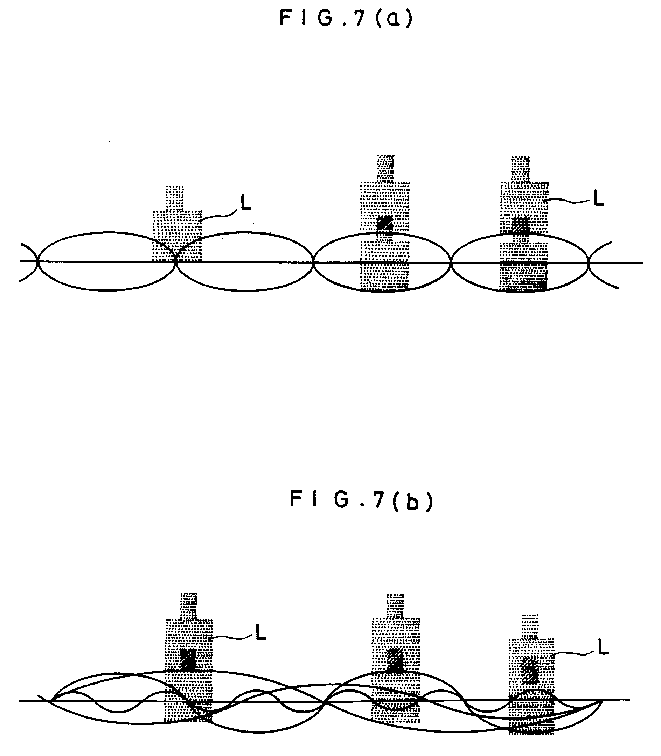

However, these vibration-generating apparatuses which use a mechanical means or ultrasonic waves produce vibrations in repetitive simple waveform and cannot change the vibration waveform. Fig. 7 (a) illustrates a vibration waveform which is formed at a single frequency by a mechanical means or by an ultrasonic oscillator, but Fig. 7 (b), on the other hand, illustrates a vibration waveform which changes constantly along with the elapse of time, as is the case with musical signals or the like. It is found that, when the vibration given in (a) is applied to a load L (a container) placed on a shelf or the like to put it into vibration, it is possible to vibrate the load L considerably in the part at the peak of the vibration amplitude, but that it is not at all possible to vibrate the load L in the part at the bottom of the vibration amplitude. On the other hand, it is evident that the vibration shown in (b) can vibrate the load L uniformly since the vibration changes moment by moment both in its frequency and in its amplitude.

The present invention has been made through utilization of this property which low frequency signals such as music have, and the method according to the present invention is thus intended for achieving improvements on the taste of certain fermented foods by giving vibrations generated with low frequency signals such as music from acoustic equipment in the process of fermentation as applied to such foods.

### SUMMARY OF THE INVENTION

As a means of overcoming the problems described above, the present invention, as defined in claim 1 below, has established a method of producing certain fermented foods, wherein the method, which is applicable to the fermentation of foods to be fermented as filled in a container or with the container or the food itself placed on a shelf, is characterized by installing electro-mechanical vibration transducers which generate vibrations with low frequency signals such as music on the container or the shelf on which the container mentioned above or the food itself and vibrating the container or the shelf with low frequency signals such as music fed to the electro-mechanical vibration transducers.

With the method according to the present invention, which is thus constituted, it is possible to give the so-called "1/f fluctuation" (to be described later), which is inherent in low frequency signals such as music, to the foods to be fermented by way of a container or a shelf in the process of fermentation and it is thereby possible to increase the activity of yeasts or the like. By this, it becomes possible to achieve a reduction of the acting period of those yeasts and also to make considerable improvements on the taste of the fermented foods.

### BRIEF DESCRIPTION OF THE DRAWINGS

Fig. 1, Fig. 3, and Fig. 4 present perspective views respectively illustrating the state in which the method according to the present invention is applied to a shelf;
Fig. 2 presents a sectional view illustrating one example of an electro-mechanical vibration transducer;
Fig. 5 and Fig. 6 respectively present perspective views illustrating a state in which the method according to the present invention is applied to a barrel and a pail;
Fig. 7 (a) and Fig. 7 (b) present illustrative drawings illustrating the relationship between the waveforms of signals and the vibrations;
Fig. 8 presents an illustrative diagram illustrating the dynamic structure of water;
Fig. 9 and Fig. 10 present charts respectively showing the state in which ethanol molecules are present in mixture with the molecules of water shown in Fig. 8; Of these charts, Fig. 9 illustrates the state of an example of Japanese sake in its freshly brewed state while Fig. 10 illustrates the state of an example of matured sake.

### DETAILED DESCRIPTION OF THE INVENTION

### Examples of Preferred Embodiments of the Invention

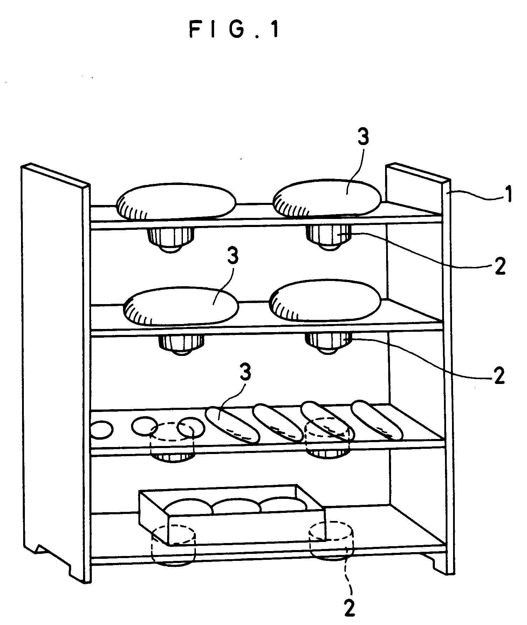

Now, a detailed description will be made of examples of preferred embodiments of the method according to the present invention. In Fig. 1, the reference number 1 indicates a shelf, which has an electro-mechanical vibration transducer 2 installed on the lower surface of each stage. On this shelf 1, bread 3 is placed in the first and second processes of fermentation. A bread baking method in common practice is as described below. The bread 3 is prepared by adding water and yeast to flour, and, after these are kneaded together thoroughly, the raw bread is left for aging for a while to wait for the completion of the first process of fermentation. After the first process of fermentation, the raw bread is formed into a predetermined shape and subjected to the second process of fermentation. Upon the completion of the second process of fermentation, the bread thus fermented is baked in an oven, from which the baked bread is taken out as a finished product in due course of time. For the application of the method according to the present invention to the baking of bread, bread is placed on the shelf 1, with low frequency vibrations such as those of musical signals being applied to the bread by the operation of electro-mechanical vibration transducers for the period of time from the state of being left for its aging to the completion of the second process of fermentation. Now that the vibrations thus applied are low frequency vibrations generated with musical signals or the like, the so-called "1/f fluctuation" characteristics (to be described later) activate the yeast, thereby achieving a reduction of the bread-producing time and an improvement on the taste of the bread.

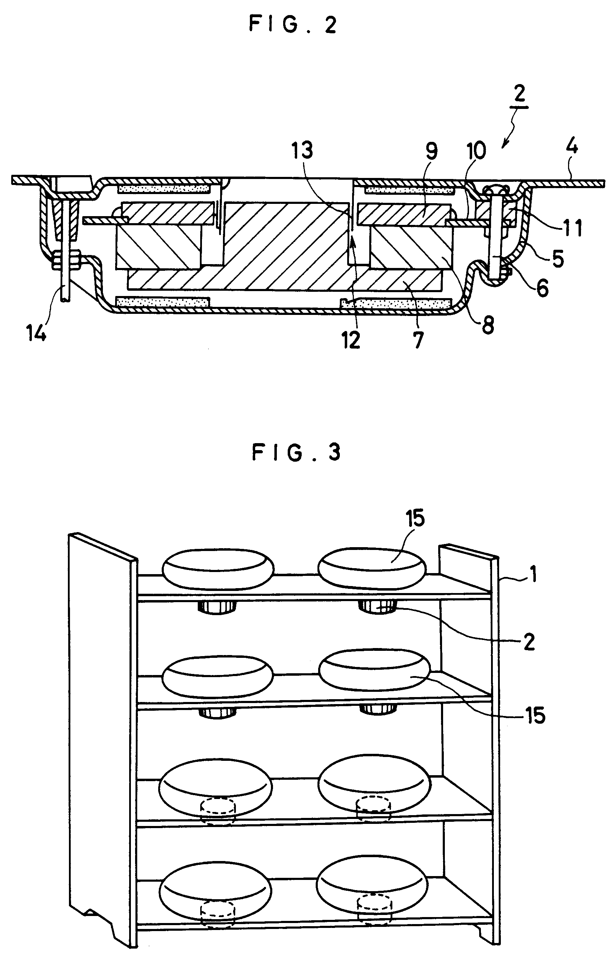

Now, an electro-mechanical vibration transducer is described with reference to Fig. 2. The electro-mechanical vibration transducer 2 has an internal construction as shown in Fig. 2. In this Fig. 2, the reference number 4 indicates a base plate the outer circumference of which is formed into a flange. The reference number 5 indicates a cover. The cover 5 is fixed by a bolt 6 on the base plate 4. Then, inside the main unit of the case formed with these, a construction as described below is accommodated, and a electro-mechanical vibration transducer 2 is thereby formed.

In Fig. 2, the reference number 7 indicates a yoke, and, as illustrated in the drawing, the yoke has a sectional shape in the form of the letter T. A ring-shaped magnet 8 is mounted on the outer circumferential area of the yoke 7, and, on the outer side thereof, a top plate 9 is provided in its contact with the magnet 8. These are supported with the above-mentioned bolt 6 via a ring spring 10 working as a damper. The reference number 11 indicates a spacer. With the construction described above, a magnetic gap 12 will be formed between the inner circumference of the top plate 9 with one surface held in contact with the magnet 8 and the T-shaped leg of the yoke 7. In the inside of this gap 12 is provided a coil 13, to which a low frequency current is fed through a cord 14 from an acoustic equipment not shown in the drawing.

The electro-mechanical vibration transducer 2 constructed in this manner receives low frequency signals such as music fed to the vibration transducer 2 in its state as installed on the shelf 1. That is to say, an integrated structure comprised of the yoke 7, the magnet 8, and the top plate 9 vibrates round the bolt 6 as the base part in relation to the coil 13 by the effect of the magnetic interference which takes place between the coil 13 and the magnet 8 when the coil 13 receives a low frequency current from an acoustic equipment not illustrated in the drawing. By this, the shelf 1 on which this electro-mechanical vibration transducer 2 is installed will vibrate together via the ring spring 10, so that also the bread 2 will vibrate. This vibration makes the activities of the yeast more active, thereby achieving a promotion of the process of fermentation and an improvement on the taste of the bread 2.

The construction shown in Fig. 3 is to be used in a case where musical vibrations are to be applied to the maturing process for cheese. In order to produce cheese 15, lactic acid bacteria and curding enzyme are added to milk, and curd is thereby produced. Then, whey (milk serum) is extracted at the same time as the curd is cut, and then the curd is shaped and compressed, and, with a weight placed on the curd and with the addition of salt to it, the curd is matured into cheese. Cheese is finished upon the completion of the maturing process. For the application of the method according to the present invention to the process of manufacturing cheese 15, which is performed in this manner, the cheese 15 is to be placed on the shelf 1 from the stage of its shaping and compression to the finish of its production. By this, low frequency vibrations such as those of music will be given to the cheese 15 placed on each shelf, and the process of maturing the cheese will attain progress in favorable condition, achieving a further improvement on its taste.

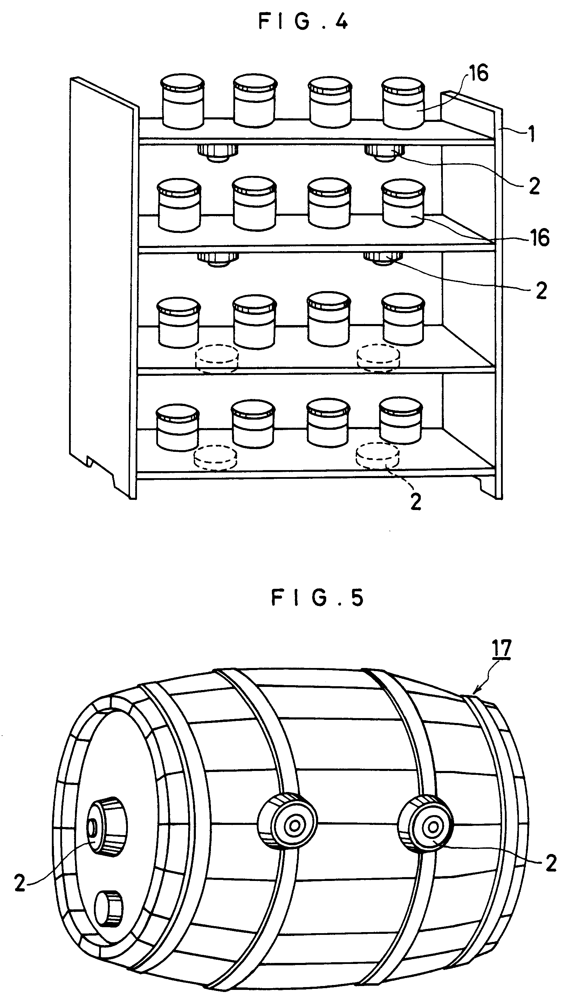

The construction shown in Fig. 4 is to be used in a case where low frequency vibrations generated by music or the like are to be applied to the process of maturing yogurt. In order to produce yogurt 16, sugar and gelatin are added to milk. Next, the milk thus prepared is heated up to approximately 90 °C, and then plain yogurt is added to the heated milk. The mixture is then filled in a bottle and cultured with its temperature maintained at approximately 40 °C. Thereafter, the cultured Yogurt is cooled and completed. For the application of the method according to the present invention to the process of manufacturing yogurt 16 as performed in this manner, the cultured yogurt is to be placed on the shelf 1 from the time of its bottling to the completion of its cooling, with the electro-mechanical vibration transducer 2 being kept in operation. By this, the low frequency vibrations generated by music or the like will be given to the yogurt 16, and the maturation of the yogurt 16 will therefore be kept in a favorable condition, and its taste will be improved.

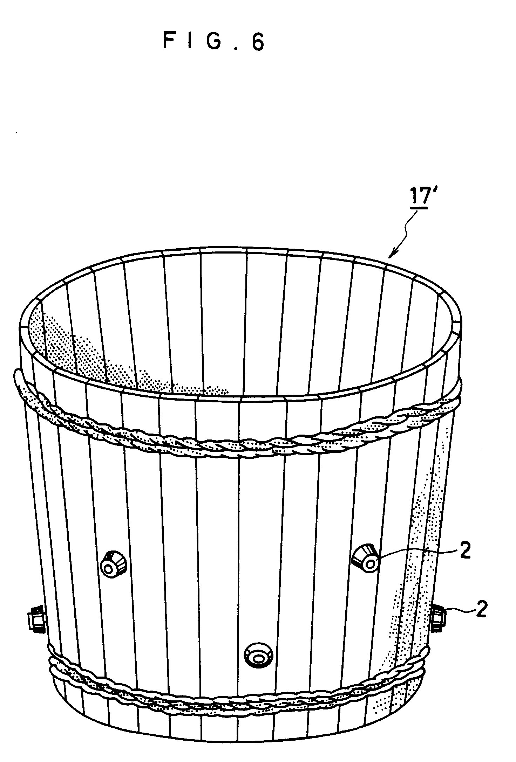

The constructions shown in Fig. 5 and in Fig. 6 are a barrel 17 and a pail 17', which are intended for the process of maturing soy bean paste, soy sauce, or picklings or the like. It is a fact known to the general public that the taste of these foods is improved by their maturation as kept in the barrel 17 or the pail 17' over a long period of time, but the feature unique to the present invention consists in giving low frequency signals such as music to these foods with an electro-mechanical vibration transducer 2 installed on the barrel 17 or the pail 17'. When the electro-mechanical vibration transducer 2 is put into action, the contents in the barrel 17 or the pail 17' will be vibrated with the low frequency signals such as music, and accordingly the process of their maturation will be made more active, so that an improvement can be achieved thereby on the taste of the foods contained in the barrel 17 or the pail 17'.

Here, a description is made of the process of giving musical signals to the electro-mechanical vibration transducer 2. In many cases, music has the characteristics of the "1/f fluctuation" having correlation to moderate changes. In this regard, the "1/f fluctuation" is used to represent the phenomenon of the measured values fluctuating around a certain mean value when various physical quantities are measured in physics. For human beings, "the 1/f fluctuation" is known as a pleasant stimulus with high affinity. A stimulus without any change in it tends to produce a weaker and weaker stimulative effect along with the elapse of time. On the other hand, a stimulus which has appropriate changes accompanying it can maintain its stimulative effect. It is known that "the 1/f fluctuation" is at work at the level of cells and also at the level of water molecules (Professor Toshimitsu Musha at the Tokyo Institute of Technology), and, generally speaking, "the 1/f fluctuation" is found to be present in all the pacifying items existing in nature and can therefore be considered to be a phenomenon with a high degree of affinity to human beings. Thus, from a scientific viewpoint, it is considered effective to use music having the characteristics of the 1/f fluctuation as a vibrating stimulus to the water molecules, the yeast, or the like. On the basis of this theory, it is possible to explain the effect of giving musical vibrations to the fermented foods.

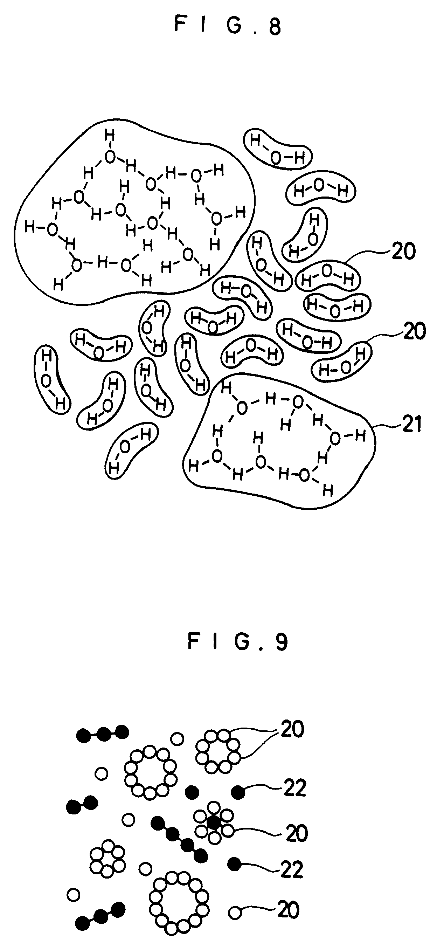

Next, the mechanism by which the taste of a food is improved with vibrations given thereto is described with reference to Japanese sake cited as examples and shown in Fig. 8 through Fig. 10. Japanese sake, or liquor for that matter, is composed of molecules of ethyl alcohol (hereinafter called ethanol) mixed and combined with the molecules of water. More specifically, the ethanol ingredient obtained from grains or fruits is mixed into selected water in good quality, and the mixture is matured. It can be considered that sake giving a well-rounded taste is produced when the molecules of ethanol thus get into the hollow holes in a cluster which is a combined unit (lump) of the molecules of water. It may be considered that this mechanism applies in the same way to such non-liquor foods as soy sauce, and an improvement can be achieved on the taste of such a food as the molecules of the basic ingredient of the food get into the hollow holes in the cluster of water.

Now, this mechanism is described with reference to the accompanying drawings. Fig. 8 illustrates a dynamic structure of water. It is considered that water, H₂O, which is composed of hydrogens combined with oxygen as seen in the molecular structure of water, does not have its molecules present in single molecules, one being isolated from another, when the water is in its liquid phase, but has a dynamic structure in which several of the water molecules 20 are formed into a cluster 21 by the force of a hydrogen bond at work among molecules. Also, it is known that the structure of water like this undergoes constant changes and that it is for a moment that a given condition is maintained.

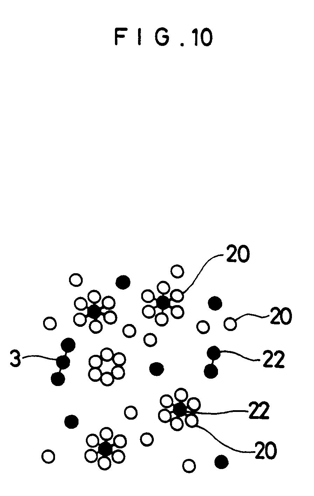

Fig. 9 and Fig. 10 illustrate the state in which the ethanol molecules 22 are present in their mixture with the molecules of water 20 in the molecular structure described above, and, of these drawings, Fig. 9 represents a case of freshly brewed liquor while Fig. 10 represents a case of a matured liquor. As it is clear from Fig. 9, the water molecules 20 and the ethanol molecules 22 are mostly isolated in the case of freshly brewed liquor, but, in the matured liquor shown in Fig. 10, the number of the ethanol molecules 22 which have entered into the void holes in the cluster 21 of the water molecules 20 grows extremely large. In this Fig. 10, the water molecules 20 which surround the ethanol molecules 22 form the cluster 21 described with reference to Fig. 9, with a large number of the water molecules 20 coming together, and an ethanol molecule 22 gets into the inside of this cluster 21 and is contained therein.

In order that an ethanol molecule 22 may get into the void hole in the water cluster 21, it is effective, as described above, to vibrate the container containing a mixture of water and ethanol continually or periodically in the course of brewing. It may be regarded that the restacking of barrels in practice at wineries is intended for the same purpose. Now, there arises a question how the vibrations are to be given. As regards the magnitude of such vibrations, even small vibrations will be able to achieve a sufficient effect, as it has already been verified through experiments on ultrasonic waves.

The present invention has been made on the basis of the theory described above. Moreover, in the examples of preferred embodiments given above, the vibration transducer having the internal structure shown in Fig. 2 is used as the electro-mechanical vibration transducer 2, but the present invention is not limited to this, and also a vibration transducer in a different construction may be used for the intended effect. In essence, a vibration transducer will be acceptable for the purpose of the present invention so long as it is capable of generating vibrations corresponding to the signals with constant changes in frequency and in amplitude, as is the case with musical signals. Moreover, it is desirable to change the output or to adjust the phase of the low frequency current which is to be applied to the electro-mechanical vibration transducer 2, depending on the size or the condition of the shelf 1 or the barrel 17.

As described above, the present invention provides a method of producing a fermented food by a process of fermenting foods with a container placed on the floor or with a container or such a food itself placed on a shelf, wherein the method is characterized by providing electro-mechanical vibration transducers which generate vibrations with low frequency signals such as music, installing the above-mentioned electro-mechanical vibration transducers either on the container mentioned above or on the shelf on which the above-mentioned container or the food itself is placed, and feeding low frequency signals such as music to the above-mentioned electro-mechanical vibration transducers.

As this method is capable of giving the so-called "1/f fluctuation", which low frequency signals such as music have in them, to the foods by way of the shelf or the barrel, as shown in the examples of preferred embodiments given above. Therefore, it is possible for the method according to the present invention not only to increase the activity of the yeast in such foods, but also to improve the taste of such foods, acting on the molecules of water contained in such foods. Thus, the method can achieve a reduction of the cultivating period for the yeast and can also make remarkable improvement of the fermented foods.

## Claims

1. A method of producing a brewed, or matured, or fermented food selected from cheese, raw bread, yoghurt and soy bean paste or picklings, by a process of brewing, maturing or fermenting the food in a container or on a shelf, by installing electro-mechanical vibration transducers for vibrating the container or the shelf in the acoustic range thereby giving vibrations to the food via the container or the shelf by feeding signals to the electro-mechanical vibration transducers:
the method being characterised by the step of:
(i) varying the amplitude and frequency of the signals with time.

2. A method according to claim 1, wherein the container defined above is a barrel or a pail.

3. A method according to claim 1 or 2, wherein the electro-mechanical vibration transducers are installed on the lower surface of the shelf.

4. A method according to claim 1, 2 or 3, wherein the food is cheese.

5. A method according to claim 1, 2 or 3, wherein the food is a raw bread.

6. A method according to claim 1, 2 or 3, wherein the food is yoghurt.

7. A method according to claim 1, 2 or 3 wherein the food is soy bean paste or picklings.

8. A method according to any one of the preceding claims wherein the signals are musical.

## Patentansprüche

1. Verfahren zur Herstellung eines gebrauten oder gereiften oder fermentierten Nahrungsmittels, ausgewählt aus Käse, Rohbrot, Joghurt und Sojabohnenpaste oder sauer Eingelegtem, durch einen Prozeß des Brauens, Reifens oder Fermentierens des Nahrungsmittels in einem Behälter oder auf einem Regal, durch Installieren von elektromechanischen Vibrationstransducern zum Vibrierenlassen des Behälters oder des Regals im akustischen Bereich, womit dem Nahrungsmittel Vibrationen über den Behälter oder das Regal vermittelt werden durch Zuführen von Signalen an die elektromechanischen Vibrationstransducer:
wobei dieses Verfahren gekennzeichnet wird durch den Schritt der:
i) Variation der Amplitude und Frequenz der Signale mit der Zeit.

2. Verfahren nach Anspruch 1, wobei der oben definierte Behälter ein Faß oder ein Bottich ist.

3. Verfahren nach Anspruch 1 oder 2, in welchem die elektromechanischen Vibrationstransducer in der unteren Fläche des Regals installiert sind.

4. Verfahren nach Anspruch 1, 2 oder 3, in welchem das Nahrungsmittel Käse ist.

5. Verfahren nach Anspruch 1, 2 oder 3, in welchem das Nahrungsmittel ein rohes Brot ist.

6. Verfahren nach Anspruch 1, 2 oder 3, in welchem das Nahrungsmittel Joghurt ist.

7. Verfahren nach Anspruch 1, 2 oder 3, in welchem das Nahrungsmittel Sojabohnenpaste oder sauer Eingelegtes ist.

8. Verfahren nach einem der vorhergehenden Ansprüche, in welchem die Signale musikalisch sind.

## Revendications

1. Procédé pour la préparation d'aliments brassés, muris ou fermentés, choisis parmi un fromage, un pain brut, un yaourt, et une pâte ou une marinade de soja, par un procédé de brassage, murissement ou fermentation des aliments dans un conteneur ou sur une tablette, par l'installation de transducteurs de vibrations électro-mécaniques pour faire vibrer le conteneur ou la tablette dans la gamme acoustique, ce qui a pour effet de conférer des vibrations aux aliments via le conteneur ou la tablette, en appliquant des signaux aux transducteurs de vibrations électro-mécaniques :
le procédé étant caractérisé par l'étape consistant à:
(i) faire varier l'amplitude et la fréquence des signaux dans le temps.

2. Procédé selon la revendication 1, dans lequel le conteneur défini ci-dessus est un tonneau ou un seau.

3. Procédé selon la revendication 1 ou 2, dans lequel les transducteurs de vibrations électro-mécaniques sont installés sur la surface inférieure de la tablette.

4. Procédé selon la revendication 1, 2 ou 3, dans lequel l'aliment est un fromage.

5. Procédé selon la revendication 1, 2 ou 3, dans lequel l'aliment est un pain brut.

6. Procédé selon la revendication 1, 2 ou 3, dans lequel l'aliment est un yaourt.

7. Procédé selon la revendication 1, 2 ou 3, dans lequel l'aliment est une pâte ou une marinade de soja.

8. Procédé selon l'une quelconque des revendications précédentes, dans lequel les signaux sont musicaux.
